# EUROPEAN PATENT APPLICATION

(11) **EP 1 642 653 A2**
(43) Date of publication of application: **05.04.2006**
(21) Application number: 05256099.2
(22) Date of filing: 29.09.2005
(51) Int. Cl.: B05D 7/24, A61L 27/34, A61L 29/08, A61L 31/10, C23C 14/12, C23C 16/50

(54) **Coated biomedical device and associated method**

(30) Priority: 30.09.2004 US 957292
(71) Applicant: DePuy Products, Inc., Warsaw, IN 46581 (US)
(72) Inventor: Grobe, George, Fort Wayne, Indiana 46845 (US); Tarr, Richard R., Winona Lake, Indiana 46590 (US); Campbell, Janine, Warsaw, Indiana 46280 (US); Paquin, Don, San Jose, California 95136 (US); Heldreth, Mark, E . Mentone, Indiana 46539 (US); Spanyer, Jonathon, Westchester, OH 45069 (US)
(74) Representative: Belcher, Simon James

(57) **Abstract**

A method of making a coated biomedical device involves generating a plasma that affects the release of a halogen species from a source. The halogen reacts with the surface of the biomedical device to form a halogen coating.

## Description

The present invention relates to biomedical devices and methods for making biomedical devices particularly devices with modified surfaces.

The medical community utilizes various biomedical devices to treat diseased and/or damaged parts of a patient's body. Examples of these biomedical devices include implantable orthopaedic devices, such as, knee, hip, shoulder, and elbow prostheses. Such orthopaedic devices typically include one or more components which are fixed to an anchoring bone by various techniques. Having the component fixed to the anchoring bone is desirable since this helps to ensure that the biomedical device will function in an appropriate manner.

A number of techniques for fixing a component of a biomedical device to an anchoring bone rely, at least in part, upon the biocompatibility characteristics of the component. One example of a biocompatibility characteristic that various fixation techniques rely upon is the osteoconductive characteristics of the component. In particular, the osteoconductive characteristics of a component contributes to its osteointegration characteristics. It should be appreciated that a component's osteointegration characteristics determines how well a bone grows around, and/or into, a component's outer surface, which typically determines how well the component is secured to the bone. In other words, the better the osteointegration characteristics of a biomedical component, the better it will be secured to the anchoring bone. Therefore, if the technique for securing a component to the anchoring bone relies, at least in part, upon the biocompatibility characteristic osteointegration, it is advantageous to improve this characteristic of the component. Accordingly, a biomedical device having one or more biocompatibility characteristics that enhance its ability to be secured to an anchoring bone is desirable. Furthermore, an associated method for producing such a biomedical device is also desirable.

A biomedical device having a coating thereon and a method of producing a coated device in accordance with the present invention comprises one or more of the following features or combinations thereof:

In one aspect, the invention provides a method for producing a biomedical device coated with a coating substance. It should be appreciated that the biomedical device to be coated may be made of any suitable material such as a metal, an alloy, an organic polymer, an inorganic material or any combination thereof In one embodiment of the invention the method for producing a biomedical device coated with a coating substance comprises, positioning the biomedical device and a solid source of the coating substance in a plasma chamber. The biomedical device and the solid source of the coating substance are then exposed to a plasma. Exposing the solid source of the coating substance to the plasma generates a reactive species of the coating substance which interacts with and binds to the biomedical device. For example, the reactive species of the coating substance may form an ionic, covalent, or coordination bond with the biomedical device and thereby cause at least a portion of the medical device to be coated with the coating substance. The coating substance may be a halogen such as F, Cl, Br, and I or mixtures thereof.

Preferably, the coating substance is fluorine. Accordingly, in this embodiment the method for producing a biomedical device coated with a coating substance comprises, positioning the biomedical device and a solid source of fluorine in a plasma chamber and exposing the biomedical device and the solid source of fluorine to plasma. As indicated above, in the presence of plasma a fluorine species is generated from the solid source of fluorine. The fluorine species interacts and binds to the biomedical device so as to dispose a fluoride coating thereon. When the coating substance is fluorine, a solid source of fluorine can comprise at least one fluoroplastic material. Illustrative examples of such fluoroplastic materials include polytetrafluoroethylene (PTFE), polyvinylidene fluoride (PVDF), polyvinyl fluoride (PVF), or a combination thereof.

In another aspect, the invention provides a method for producing a biomedical device coated with a coating substance may comprise exposing a biomedical device to a plasma generated in the presence of a mixture of a gas such as tetrafluoromethane (CF₄) and oxygen (O₂), wherein a fluorine species is generated. The fluorine species interacts with and binds to the biomedical device. For example the fluorine species interacts with and binds to a metallic surface of the biomedical device.

In yet another aspect, the invention provides a method for producing a biomedical device coated with a coating substance may comprise generating the plasma using at least one gas. The gas may include, but is not limited to, hydrogen (H₂), oxygen (O₂), air, argon (Ar), tetrafluoromethane (CF₄), or a mixture thereof. To generate the plasma, a radio frequency of, for example, about 13.56 mHz with an energy of about 100 W to about 1000 W is suitable. The plasma process may be run under a pressure of about 100 mTorr to about 500 mTorr. In a specific embodiment, the step of generating the plasma is performed using a first gas and a second gas, sequentially. The first gas may be different from the second gas. Each of the first gas and the second gas may include air, argon (Ar), hydrogen (H₂), oxygen (O₂), tetrafluoromethane (CF₄), or a mixture thereof.

The method of the present invention may include the step of cleaning the surface of the biomedical device in the chamber prior to positioning a solid source of the coating substance therein. The cleaning step may be performed by subjecting the surface of the biomedical device to an oxidizing plasma process and/or a reducing plasma process (for example 5 min H₂ followed by 30 min O₂). The oxidizing plasma may be activated in the presence of air, oxygen, carbon dioxide, or a mixture of nitrogen and oxygen. The oxidizing plasma may be activated with a radio frequency at about 13.56 mHz and with the wattage of about 100 W to about 1000 W. The chamber can be kept under a pressure of about 100 mTorr to about 500 mTorr while the oxidizing plasma process is underway.

In another aspect, the invention provides a biomedical device comprises a surface having a coating substance chemically bound thereto. For example, bound to the biomedical device via ionic and/or covalent bonds. As discussed above the coating substance may be a halogen, for example, fluorine may be chemically bound to the biomedical device. In one embodiment a fluoride coating of a surface of a biomedical device may have a fluorine content of about 6 atom % to about 53 atom %, and may be free of a solute/solvent residue.

Biomedical devices of the kind with which this invention is concerned include, for example, tissue implants or orthopaedic implants (e.g. knee, hip, shoulder, and elbow prostheses), blood contacting devices for diagnosis and/or therapy, catheters, vascular graft materials or other biomedically suitable articles. Biomedical devices may be made of any suitable biocompatible substrate such as polymers, inorganic substrate, such as hydroxyapatite, metals, metal alloys, or any combination thereof. The metal and metal alloys may include, for example, titanium, titanium alloys, stainless steel, or any cobalt- based alloys, such as CoCrMo (ASTM F1537). It is contemplated that a single biomedical device may be made of one or more components. Each component may be made of the same or different substrate material.

The invention provides a method for producing a biomedical device having a coating thereon. It is contemplated that the coating may include halogens such as F, Cl, Br, and I or a mixture of theses elements. The halogen may form ionic and/or covalent bonds with the surface of the biomedical device. The coating may consist of just one or a mixture of these halogens, or it may include other elements in addition to one or more of these halogens. It should be appreciated that the following discussion is directed to using fluorine as the coating substance. However, as indicated herein, using other halogens as the coating substance in the methods described herein is contemplated. In addition, biomedical devices having a coating thereon that includes one or more of the disclosed halogens disposed thereon are also contemplated.

With respect to disposing a fluoride coating on a biomedical device, the coating acts as an anti-microbial agent and/or promotes the attachment of tissue and/or bone to the biomedical device. The fluoride coating can also promote the growth of bone into structures formed on the surface of a biomedical device (e.g. pores, depressions, or any other surface structure formed on the surface of a biomedical device which bone is able to grow into). One aspect of the present invention involves placing a biomedical device to be coated in a plasma chamber and then creating a plasma in the chamber in the presence of a solid source of fluorine. A reactive species in the plasma interacts with the solid source of fluorine which results in a fluorine species being released from the solid source of fluorine. The fluorine species released from the solid material, such as fluorine ions or free radicals, react with, for example, the surface of the biomedical device to form a fluoride coating thereon. For example, fluorine released from the solid source of fluorine covalently bonds to the surface of the biomedical device to form the fluoride coating.

An example of a preferred fluorine source is a fluorine containing solid, such as a fluoroplastic material. However, it should be appreciated that any fluorine containing solid can be utilized in the present invention as long as it releases a fluorine species which is able to coat a biomedical device when exposed to a plasma under the appropriate conditions. In addition, it should be understood that the use of a solid source of fluorine is advantageous over a gas source because most fluorine containing gas (F₂, CF₄, etc.) can pose health and environmental risks. In addition, gaseous fluorine sources are more difficult to handle, store, and manipulate than a solid source of fluorine. The fluoroplastic material that may be used in the present method includes, but is not limited to, polytetrafluoroethylene (PTFE such as that sold under the trade mark TEFLON), polyvinylidene fluoride (PVDF such as that sold under the trade mark KYNAR), and polyvinyl fluoride (PVF such as that sold under the trade mark TEDLAR). These materials are available in various forms, sizes and dimensions. For example, PTFE is available as a plumbing tape, a ring, a ball, or a block. PVF is available in a wide variety of films and resins. With respect to a solid source of other halogens, e.g. Cl, Br, and I, other solid sources can be utilized. For example, chlorinated, brominated, and/or iodinated solid polymers may be used as the solid source of the coating substance. In particular, an illustrative example of a solid source of the halogen Cl is polyvinyl chloride (PVC). As previously mentioned, the handling of a solid source of a halogen, such as fluorine, is easy and convenient. For instance, a PTFE tape can be cut into suitable sizes and positioned in various ways within a plasma chamber relative to the biomedical device or article to be coated.

The present invention contemplates the use of fluorine sources that are not solid. For example, it is contemplated that CF₄ or F₂ gas, alone or a mixture with another gas, such as oxygen (O₂), may also be used in coating a biomedical device. It has been found that a CF₄/O₂ mixture at a ratio of about 90 parts of CF₄ to about 10 parts of O₂ is effective in the process of plasma-induced fluoride coating of a biomedical device. In addition, it is contemplated that fluorine sources that are not solid can be utilized in conjunction with a solid source of fluorine to generate fluorine species to interact with the biomedical device.

With respect to the "plasma process" mentioned above, this refers to a cold (non- equilibrium) gas plasma initiated by a low energy discharge. The plasma process may be understood as an energy dependent process involving energetic gas molecules. Collisions between energetic gas molecules cause the formation of a reactive species or the "plasma", which includes ions, excited molecules, and free radicals of the gas. The gas used to generate the plasma may be any suitable gas that produces a reactive species which is capable of releasing a reactive fluorine species from a solid source of fluorine. Examples of suitable gases are hydrogen (H₂) and oxygen (O₂). It is also possible to use an inert gas such as argon (Ar) as the plasma source. In addition, other reactive gasses such as tetrafluoromethane (CF₄) or other halogen gases may also be used. In addition, it is contemplated that a sequential supply of at least two different gasses or a mixture of gasses may be used to produce the plasma of the present invention. For example H₂ may be used first to generate a plasma during the initial phase, and then H₂ may be replaced by O₂ to generate the plasma for a subsequent phase.

The plasma may be generated using any suitable energy, such as electrical discharges, direct currents, microwaves or other forms of electromagnetic radiation. The energy used in the examples described below is a radio frequency energy. The radio frequency of about 13.56 mHz is particularly suitable. The wattage of the energy may vary between about 100 W and about 1000 W. A range from about 300 W to about 500 W is particularly effective. The plasma process should be performed under a pressure of between about 20 mTorr and about 8500 mTorr. A pressure in the range of about 100 mTorr to about 500 mTorr is effective. Particularly, a pressure of 300 mTorr is often used in the illustrative examples. The conditions and the duration of the plasma treatment may be varied as needed depending on factors such as the gas used to generate the plasma, the halogen source (e.g. the fluorine source) the amount of coating desired, and the type of biomedical material to be coated.

The plasma process of the present invention may be performed using any suitable plasma chamber/system. Many suitable plasma systems are commercially available. For example, Technics Plasma Etch IIB System (Technics, Phoenix Arizona), and TePla 7200 Series Plasma System (PVA TePla America, Inc., Corona, CA) can be utilized in the present invention. These systems are provided with manufacturer's instructions which are fully incorporated herein by reference.

It should be appreciated that the plasma chamber should be free of any contaminant prior to the start of coating a biomedical device as described herein. Chamber cleaning/decontamination techniques are well known in the art. In addition, the biomedical device to be coated should also have its surface cleaned prior to initiating the process. It is well known in the art that a plasma process may be used for the purpose of surface cleaning. The plasma process for the purpose of cleaning usually utilizes an oxidizing plasma gas such as oxygen or carbon dioxide to remove adventitious carbon contaminants. It is contemplated that air or a mixture of nitrogen and oxygen can also be used. It is further contemplated that non-oxidizing plasma, using an inert gas or an inert gas mixture or H₂ may also be used for cleaning purposes.

Preferably, the plasma for surface cleaning is activated using a radio frequency of about 13.56 mHz, at the wattage of about 100 W to about 1000 W. The pressure of the plasma chamber should be kept at between about 100 mTorr to about 500 mTorr. The duration of the plasma cleaning may vary as needed. About 10 to about 60 minutes, and typically about 35 minutes, of the plasma cleaning process is sufficient to purge the surface contaminants depending on pressure, power, and gas used. It is contemplated that the cleaning plasma process may be performed immediately before the fluoride coating treatment, in the same plasma chamber.

After the biomedical device has been cleaned as described above and is ready to be coated, a solid source of fluorine may be placed in the plasma chamber. The solid source of fluorine should be positioned in close proximity to the biomedical device. In addition, the solid source of fluorine should be positioned in the chamber so that it is located between the gas inlet of the plasma chamber and the biomedical device. In other words the solid source of the fluorine should be located upstream relative to the biomedical device.

Embodiments of the invention will now be described by way of example with reference to the accompanying drawings, in which:
Figure 1 is an illustrative diagram of a top view of a plasma chamber during a fluorine treatment according to an exemplary embodiment of the present invention; and
Figure 2 is an illustrative diagram of a front view of a plasma chamber during a fluorine treatment according to another exemplary embodiment of the present invention.

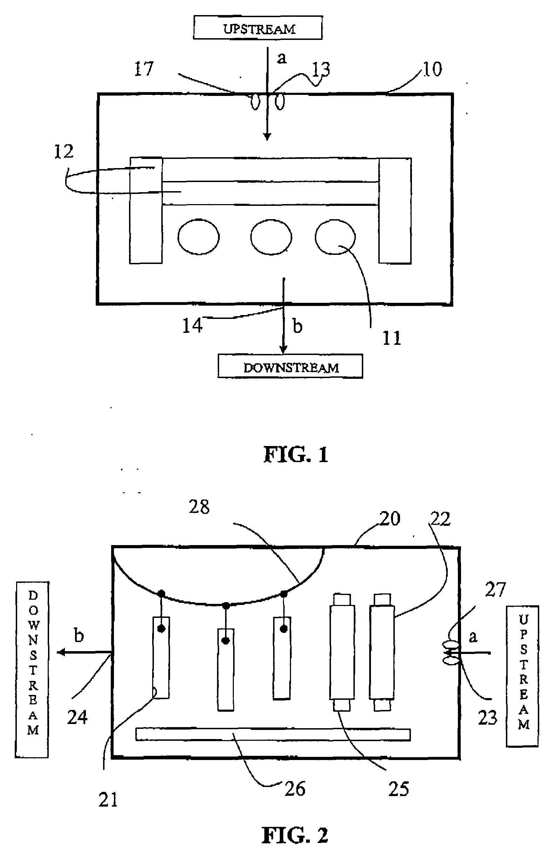

As demonstrated in Figure 1, one or more biomedical devices 11 (represented as a plurality of circles) is positioned downstream relative to a solid source of fluorine 12, within plasma chamber 10. Solid source of fluorine 12 is generally configured in a U-shape to allow biomedical devices 11 to be sufficiently exposed to solid source of fluorine 12. It is contemplated that solid source of fluorine 12 may be formed in any arrangement as long as at least one side of biomedical device 11 is surrounded by or exposed to solid source of fluorine 12. Gas inlet 13 is positioned upstream of solid source of fluorine 12. Gas outlet 14 is positioned downstream of biomedical device 11. In operation, when the gas is discharged into chamber 10 through gas inlet 13 in the direction of arrow a, excited reactive plasma is formed by the gas species activated by the energy discharged via electrodes 17. The reactive plasma is driven towards solid source of fluorine 12 to react with the surface of solid source of fluorine 12, causing a release of fluorine species (e.g. ions and/or free radicals) from solid source of fluorine 12. The released fluorine species are subsequently driven towards biomedical devices 11 to react with the surface substrate of biomedical devices 11, forming chemical bonds between the fluorine species and the surface of biomedical devices 11. The excess gas subsequently exit plasma chamber 10 through gas outlet 14 in the direction of arrow *b*.

In another example, as demonstrated in Figure 2, one or more solid sources of fluorine 22 are provided in plasma chamber 20. Each solid source of fluorine 22 is wrapped around support article 25, which may be a glass slide or a glass block. Each biomedical device 21 is suspended from wire 28, which may be a nickel plated steel wire, over tray 26, which may be aluminum. Each solid source of fluorine 22 is positioned downstream of gas inlet 23, and upstream of each biomedical device 21. When the gas enters plasma chamber 20 through gas inlet 23 in the direction of arrow a, a radio frequency energy is provided via electrodes 27 to produce excited reactive plasma species. The excited reactive plasma species are driven toward each solid source of fluorine 22 to react with the surface of solid source of fluorine 22 and cause a release of a fluorine species (e.g. ions and/or free radicals) from solid source of fluorine 22. The released fluorine species, in turn, are driven towards biomedical each device 21 to react with the surface of each biomedical device 21, forming chemical bonds between the fluorine species and the substrate surface of each biomedical device 21. The excess gas subsequently exits plasma chamber 20 through gas outlet 24 in the direction of arrow b.

Once the fluoride coating treatment as above-described is completed, the resulting coated biomedical device may be surface analysed using X-ray photoelectron spectroscopy (XPS) to determine the surface content of fluorine and other elements. The XPS measures elemental and chemical composition over approximately the outermost 100 Angstroms of a specimen. A commercially available XPS system that has been used is that sold under the trade mark PHI Quantum 2000 XPS by Physical Electronics USA, Chanhassen, Minnesota. It is contemplated that any other surface analysis methods may also be used to determine the surface content of different elements or compounds.

Based on the XPS analysis, the fluorine content of the treated surface may, for example, range from about 6 atom % to over 50 atom %. The presence of fluoride, not a fluorocarbon is known from analysis of C1S envelope obtained from high resolution XPS analysis. Samples having a fluoride coating disposed thereon exhibit characteristically hydrophilic surfaces, with contact angles ranging from 0° (completely wetting the surface) to 15°, where perfluorocarbon surfaces display contact angles as high as 115°.

In view of the forgoing, the plasma process of the present invention is beneficial in that the process is clean, and easy to performed. The resulting coated biomedical device is also clean and does not require rinsing or cleaning after the coating treatment.

In the examples described below, substrate coupons were used as representatives of the biomedical devices to be treated A control was run in each example which showed that each test coupon had a minimal amount of fluorine present on its surface prior to being subjected to the described procedure. The arrangements and the numbers of the devices to be treated will depend on the type, size, and the dimension of the particular biomedical devices. If the devices have more than one component, each component may be treated individually or in combination with other components. It is contemplated that appropriate adjustments may be made in accordance with the spirit of the present invention.

### Example 1 - Cleaning plasma chamber

Prior to running the experiments, the empty plasma chamber was cleaned by running three consecutive cycles of oxygen, argon, and hydrogen as the gas source. The plasma was generated using the radio frequency of 13,56 mHz at the wattage of 300 W under a pressure of 300 mTorr. The plasma was run for 10 minutes per gas.

### Example 2 - Fluoride deposition on CoCrMo (ASTM F 1537) using PTFE block as fluorine source

Using a Technics Plasma Etch IIB system, CoCrMo coupons (about 5 mm × 20 mm × 2 mm) were positioned on a PTFE block (about 3 cm × 5 cm × 15 cm) from grooves (3 mm) approximately 2.5 cm apart. The block was positioned mid-chamber. The plasma was generated with the radio frequency of 13.56 mHz at the wattage of 300 W, and under a pressure of 300 mTorr. The plasma was generated in the presence of oxygen and run for 10 minutes, followed by hydrogen for 10 minutes. The treated coupons were subjected to surface analysis using PHI Quantum 2000 XPS system. Sampling area was approximately 1.0 sq mm. The results show the following surface components (atom %): fluorine 29 ± 1 %, carbon 18 ±2 %, oxygen 27 ± 1 %, cobalt 16 ± 1 %, chromium 1, ± 0.1 %, molybdenum trace, aluminum 5 ± 1%, sodium 1 ± 0.3%, silicon nd (none detected) , phosphorus 1 ± 0.3%, sulfur nd, potassium nd, copper 1 ± 0.1%, zinc 1 ± 0.1%.

### Example 3 - Fluoride deposition on CoCrMo (ASTM F1537) using PTFE block as fluorine source.

Using a Technics Plasma Etch IIB system, CoCrMo coupons (5 mm × 20 mm × 2 mm) were perched on a TEFLON block (about 3 cm × 5 cm × 15 cm) from grooves (3 mm) approximately 2.5 cm apart. The block was positioned mid-chamber, The plasma was generated with the radio frequency of 13.56 mHz at the wattage of 300 W and under a pressure of 300 mTorr, in the presence of argon. The treatment was run for 10 min. The treated coupons were analysed as described in Example 2. The results show the following surface components (% atom): fluorine 8 ± 3%, carbon 61 ± 9%, oxygen 19 ± 3%, cobalt 6 ± 2%, chromium 3 ± 1%, molybdenum 0.4 ±0.1%, aluminum 3 ± 1%, sodium 1 ± 1 %, silicon nd, phosphorus nd, sulfur nd, potassium nd, copper trace, zinc trace.

### Example 4 - Fluoride deposition on CoCrMo (ASTM F1537) using PTFE block as fluorine source

Using a Technics Plasma Etch IIB system, CoCrMo coupons (5 mm × 20 mm × 2 mm) were positioned on a PTFE block (about 3 cm ×3 mm) approximately 2.5 cm apart. The block was positioned mid-cbamber. The plasma was generated with the radio frequency of 13.56 mHz at the wattage of 300 W and under a pressure of 300 mTorr, using a hydrogen source, for 10 min. The treated coupons were analysed as described in Example 2. The results show the following surface components (% atom): fluorine 11 ± 3%, carbon 38 ± 10%, oxygen 32 ± 4%, cobalt 7 ± 2%, chromium 2 ± 2%, molybdenum 0.5 ±0.1, aluminum 5 ± 2%, sodium 2 ± 1%, silicon 1 ± 0.2%, phosphorus 2 ± 0.5%, sulfur 2 ± 1%, potassium nd, copper 1 ± 0.01%, zinc 0.4 ± 0.1%.

### Example 5 - Fluoride deposition on CoCrMo (ASTM F 1537) using CF₄/O₂ as fluorine source

Using a Technics Plasma Etch IIB system, CoCrMo coupons (5 mm × 20 mm × 2 mm) were suspended via nickel plated steel wire above the tray (about 5 cm × 10 cm). The plasma was generated using the radio frequency of 13.56 mHz at the wattage of 300 W in the presence of an CF₄/O₂ (90/10) mix. The plasma treatment was run for 10 minutes under 300 mTorr pressure. The treated coupons were analysed as described in Example 2. The results show the following surface components (% atom): fluorine 33 ± 7%, carbon 23 ± 5%, oxygen 22 ± 5%, cobalt 9 ± 2%, chromium 1 ± 0.3%, molybdenum nd, aluminum 9 ± 2%, sodium 1 ± 1%, silicon nd, phosphorus trace, sulfur nd, potassium nd, copper I ± 0.2%, zinc 0.4 ± 0.1 %.

### Example 6 - Fluorine deposition on CoCrMo (ASTM F1537) using PTFE tape as fluorine source

Using a Technics Plasma Etch IIB system, CoCrMo coupons (5 mm × 20 mm × 2 mm) were suspended via nickel plated steel wire above the tray (5 cm × 10 cm). Two glass microscope slides (about 2.5 cm × 7.5 cm) were placed immediately upstream of the coupons. The slides were wrapped in TEFLON tape (1 mm × 13 mm) in such a way that the tape was positioned circumferentially about the long axis of the slide. The plasma was generated in the presence of hydrogen using the radio frequency of 13.56 mHz at the wattage of 300 W and was run under a pressure of 300 mTorr for 10 minutes. The treated coupons were analysed as described in Example 2. The results show the following surface components: fluorine 40 ± 3%, carbon 22 ± 3%, oxygen 15 ± 1%, cobalt 5 ± 1%, chromium 2 ± 1%, molybdenum trace, aluminum 13 ± 1%, sodium 2 ± 1%, silicon nd, phosphorus 1 ± 0.1%, sulfur trace%, potassium trace, copper 1 ± 0.1 %, zinc 1 ± 0.2%, tin nd, calcium nd, chlorine nd.

### Example 7 - Fluoride deposition on CoCrMo (ASTM F1537) using PTFE tape as fluorine source

Using a Technics Plasma Etch IIB system, CoCrMo coupons (5 mm × 20 mm × 2 mm) were suspended via nickel plated steel wire above the tray (5 cm × 10 cm). Two glass microscope slides (2.5 cm × 7.5 cm) were placed immediately upstream of the coupons. The slides were wrapped in PTFE tape (1.0 mm × 13 mm) in such a way that the tape was positioned circumferentially about the long axis of the slide. The plasma was generated using the radio frequency of 13.56 mHz at the wattage of 300 W, and run at 300 mTorr in the presence of oxygen for 10 min, followed by hydrogen for 10 min. The treated coupons were analysed as described in Example 2. The results show the following surface components (% atom): fluorine 45 ± 3%, carbon 11 ± 1%, oxygen 18 ± 1%, cobalt 6 ± 1%, chromium 1 ± 0.2%, molybdenum trace, aluminum 14 ± 2%, sodium 2 ± 0,1 %, silicon nd, phosphorus 1 ± 0.2%, sulfur 1 ± 0.1%, potassium 1 ± 0.1%, copper 2 ± 0.4%, zinc 1 ± 0.1%, tin nd, calcium trace, chlorine nd.

### Example 8 - Fluoride deposition on CoCrMo (ASTM F1537) using PTFE tape as fluorine source

Using a TePla 7200 Series Plasma System (Corona, CA), CoCrMo coupons (5 mm × 20 mm × 2 mm) were placed in the centre of the third (centre) aluminum shelf. Shelves 1, 2, 4, and 5 were removed to concentrate the plasma reaction. Four strips (about 5 cm × 10 cm × 2 cm and about 5 cm × 5 cm × 2 cm) of PTFE tape were placed around the samples on the tray. Together the four strips formed a complete square enclosing the samples. The plasma was generated with the radio frequency of 13.56 mHz at the wattage of 1000 W and under a pressure of 300 mTorr. The plasma treatment was run in the presence of hydrogen for 20 minutes, followed by oxygen for 20 minutes. After the experiment was complete, the TEFLON tape had almost completely disintegrated. There was no change in the appearance of the coupons. The treated coupons were analysed as described in Example 2. The results show the following surface component (% atom)s: fluorine 32 ± 1%, carbon 2 ± 1%, oxygen 10 ± 1%, cobalt 32 ± 1%, chromium 1 ± 0.2%, molybdenum nd, aluminum nd, gold 1 ± 0.1%, nitrogen 1 ± 0.1%, magnesium 1 ± 0.1%, zinc 1 ± 0,2%, silicon nd, tin nd, phosphorus nd, calcium nd, boron nd, copper nd, sodium nd.

### Example 9 - Fluoride deposition on polyetherimide using PTFE tape as fluorine source

Using a TePla 7200 Series (Plasma System, Corona, CA), coupons made from a polyetherimide resin (such as that sold by GE Plastics under the trade mark ULTEM), approximately 2.54 cm in diameter and 2.54 cm in height, were placed in the centre of the third (centre) shelf. Shelves 1, 2, 4, and 5 were removed. The plasma was activated by the radio frequency of 13.56 mHz at the wattage of 1000 W. The plasma treatment was run under a pressure of 300 mTorr using hydrogen for 20 minutes and followed by oxygen for 20 minutes. The results show the following surface component (% atom)s: fluorine 7 ± 1 %, carbon 60 ± 1 %, oxygen 26 ± 1%, nitrogen 4 ± 0.2, sodium 1 ± 0.4, Si trace, Ca nd, Cl nd, Al2 ± 0.2, Ti 1 ± 0.1, Mg trace.

### Example 10 - Fluoride deposition on a crystalline hydroxyapatite using PTFE tape as fluorine source.

The hydroxyapatite consisted of a layered composite with hydroxyapatite (about 3-6 microns thick) deposited onto disks of Ti-6Al-4V (1.0 mm thick × 9 mm) sintered with 3 mm thick titanium (cp) beads. Using a TePla 7200 Series (Plasma System, Corona, CA), the apatite-Ti coupons were placed in the centre of the third (centre) aluminum shelf and surrounded on 4 sides with PTFE tape. Shelves 1, 2, 4, and 5 were removed. Coupons were positioned downstream of the plasma using hydrogen for 20 minutes and followed by oxygen for 20 minutes. The plasma was activated by the radio frequency of 13.56 mHz at the wattage of 1000 W. The plasma treatment was run under a pressure of 300 mTorr. The results show the following surface component (atom %): fluorine 40 ± 2%, carbon 3 ± 0.4%, oxygen 34 ± 2%, titanium 3 ± 1%, aluminum 5 ±1%, nitrogen trace, magnesium 0.6 ± 0.01%, zinc nd%, phosphorus 4 ± 5%, calcium 10 ± 1 %, boron nd, copper nd.

## Claims

**1.** A method for producing a fluoride coated biomedical device, comprising exposing a surface of a biomedical device to a plasma in the presence of a solid source of fluorine, in which a fluorine species is generated from the solid source of fluorine and interacts with the surface of the biomedical device.

**2.** The method of claim 1, in which the plasma is generated using at least one of hydrogen (H₂), oxygen (O₂), air, argon (Ar), and tetrafluoromethane (CF₄).

**3.** The method of claim 1, in which the plasma is activated using a radio frequency of 13.56 mHz at the wattage of about 100 W to about 1000 W.

**4.** The method of claim 1, which is performed under a pressure of about 100 mTorr to about 500 mTorr.

**5.** The method of claim 1, in which the surface of the medical device is made of a material selected from the group comprising: a metal, an alloy, an organic polymer, and an inorganic material.

**6.** A method of making a fluoride coated biomedical device, comprising:
positioning a biomedical device having a surface in a plasma chamber,
positioning a solid source of fluorine in the chamber,
generating a plasma in the plasma chamber,
reacting the plasma with the solid source of fluorine to generate fluorine species, and
reacting the fluorine species with the surface of the biomedical device.

**7.** The method of claim 1 or claim 6, in which the solid source of fluorine comprises at least one fluoroplastic material.

**8.** The method of claim 7, in which one fluoroplastic material is polytetrafluoroethylene (PTFE), polyvinylidene fluoride (PVDF), or polyvinyl fluoride (PVF).

**10.** A biomedical device comprising a surface having fluorine chemically bound thereto.

**11.** The biomedical device of claim 10, in which the surface has an atom % of fluorine of about 6 atom % to about 53 atom %.

**12.** The biomedical device of claim 11, in which the surface is a metal, a metal alloy, an organic polymer, or an inorganic material.

**13.** The biomedical device of claim 10, in which fluorine forms a coordination bond.

**14.** A biomedical device comprising a surface having a halogen chemically bound thereto, in which the surface has an atom % of the halogen of about 6 atom % to about 53 atom %.

**15.** A method of treating a biomedical device, comprising:
positioning a biomedical device having a surface in a plasma chamber;
positioning a solid source of a halogen in the chamber;
generating a plasma in the plasma chamber;
reacting the plasma with the solid source of the halogen to generate a halogen species, and
exposing the surface of the biomedical device to the halogen species.

**16.** The method of claim 15, in which the halogen species chemically binds to the surface of the biomedical device.

**17.** The method of claim 15, in which subsequent to exposing the surface of the biomedical device to the halogen species the surface has an atom % of the halogen of about 6 atom % to about 53 atom %.
